# FASCICULE DE BREVET EUROPEEN

(11) **EP 2 758 521 B1**
(45) Date de publication et mention de la délivrance du brevet: **26.08.2015**
(21) Numéro de dépôt: 12773050.5
(22) Date de dépôt: 17.09.2012
(51) Int. Cl.: C12N 5/00

(54) **DISPOSITIF DE GUIDAGE DE LA MIGRATION CELLULAIRE ET PROCEDE DE GUIDAGE DE LA MIGRATION CELLULAIRE METTANT EN OEUVRE UN TEL DISPOSITIF**
VORRICHTUNG ZUR FÜHRUNG EINER ZELLMIGRATION UND VERFAHREN ZUR FÜHRUNG EINER ZELLMIGRATION MITHILFE DIESER VORRICHTUNG
DEVICE FOR GUIDING CELL MIGRATION AND METHOD OF GUIDING CELL MIGRATION IMPLEMENTING SUCH A DEVICE

(30) Priorité: 19.09.2011 FR 1158317
(43) Date de publication de la demande: 30.07.2014
(73) Titulaire: Institut Curie, 75005 Paris (FR); Centre National de la Recherche Scientifique (CNRS), 75016 Paris (FR); Société de Developpement et de Recherche Industrielle, 21300 Chenove (FR)
(72) Inventeur: LE BERRE, Maël, F-75020 Paris (FR); PIEL, Matthieu, F-75012 Paris (FR); LIU, Yanjun, F-92400 Courbevoie (FR)
(74) Mandataire: Cabinet Plasseraud
(86) Numéro de dépôt international: PCT/FR2012/052077
(87) Numéro de publication internationale: WO 2013/041803

(56) Documents cités:
- US-A1- 2009 248 145
- GOHER MAHMUD ET AL: "Directing cell motions on micropatterned ratchets", NATURE PHYSICS, vol. 5, no. 8, 1 août 2009 (2009-08-01), pages 606-612, XP055034675, ISSN: 1745-2473, DOI: 10.1038/nphys1306
- HWA SENG KHOO ET AL: "Engineering the 3D architecture and hydrophobicity of methyltrichlorosilane nanostructures", NANOTECHNOLOGY, IOP, BRISTOL, GB, vol. 19, no. 34, 27 août 2008 (2008-08-27) , page 345603, XP020144505, ISSN: 0957-4484, DOI: 10.1088/0957-4484/19/34/345603
- SE YON HWANG ET AL: "Adhesion Assays of Endothelial Cells on Nanopatterned Surfaces within a Microfluidic Channel", ANALYTICAL CHEMISTRY, vol. 82, no. 7, 1 avril 2010 (2010-04-01), pages 3016-3022, XP055034676, ISSN: 0003-2700, DOI: 10.1021/ac100107z

## Description

L'invention se rapporte à un dispositif de guidage de la migration cellulaire et à une méthode de guidage de la migration cellulaire mettant en oeuvre un tel dispositif.

En particulier, l'invention se rapporte à un dispositif de guidage de la migration cellulaire comprenant une surface support pourvue de cellules et un substrat, ledit substrat présentant une surface texturée placée en regard de la surface support et mise en contact avec les cellules placées sur la surface support.

La migration des cellules est essentielle pour de nombreux processus physiologiques comme l'organogénèse ou la cicatrisation des plaies. Dans leur environnement naturel, la direction et la vitesse de migration des cellules sont guidées par de nombreux signaux qui peuvent être chimiques (chimiokines) ou physiques (microenvironnement).

In vitro, ces phénomènes peuvent être reproduits ou détournés pour imposer une direction de migration aux cellules en utilisant, par exemple, des chemoattractants, des champs électriques ou encore en modulant l'environnement mécanique de la cellule.

Le document EP-A-1199354 décrit, par exemple, la formation d'un motif de cellules sur une surface par un contrôle chimique de la migration des cellules. En effet, dans le document EP-A-1199354, la surface est traitée de manière à présenter un pré-motif constitué de composés favorisant le développement des cellules et d'autres composés ne favorisant pas le développement des cellules. La culture des cellules est ensuite initiée sur ce pré-motif. Cependant, l'efficacité du contrôle de la migration des cellules par ce type de système dépend principalement du choix des composés chimiques favorisant ou empêchant le développement cellulaire en fonction de la nature des cellules cultivées.

Le document US 2007/0009572 décrit, quant à lui, une méthode de préparation d'un film biodégradable micro- ou nano-texturé comprenant des canaux dont la largeur peut varier de 10 µm à 160 µm, sur lequel sont déposées des cellules musculaires. Les essais réalisés montrent que les cellules musculaires s'alignent les unes par rapport aux autres le long des canaux, et que leur morphologie se modifie pour prendre une forme allongée. Cette méthode n'a pas pour finalité de faire migrer les cellules dans une direction préférée mais juste de favoriser leur alignement les unes par rapport aux autres pour obtenir un empilement cellulaire régulier.

Le document US 2009/02481445 décrit également une méthode pour guider l'orientation des cellules suivant une structure tridimensionnelle en utilisant une surface comprenant un micro-canal ou une série de micro-canaux parallèles les uns aux autres, dont la largeur est supérieure à celle des cellules afin que les cellules puissent s'y introduire et dont la section est arbitraire. Comme pour le document précédent, cette méthode n'a pas pour finalité de faire migrer les cellules dans une direction préférée, mais juste de favoriser leur alignement les unes par rapport aux autres.

Mahmud et al. (Nature Physics 2009, 4, pp. 606) proposent des motifs adhésifs en forme de cliquets (« ratchet ») pour guider la migration cellulaire. L'effet observé est basé sur un contraste d'adhésion entre les parties adhésives des canaux et les parties non-adhésives d'un substrat de sorte que, lorsque la qualité du contraste entre les parties adhésives et non-adhésives se dégrade avec le temps, le guidage de la migration cellulaire n'est plus observé. En outre, l'adhésion sur les canaux, linéaires ou présentant une forme de cliquets (« ratchet »), ne permet de maintenir les cellules que sur ces motifs adhésifs, c'est-à-dire sur une seule dimension dans l'espace, et ne permet pas, par exemple, l'organisation d'un tissu sur une surface en deux dimensions. Enfin, les motifs décrits dans Mahmud *et al.* sont toujours des projections perpendiculaires au plan constitué par la surface véhiculant les cellules.

Ces méthodes permettant de détourner les phénomènes naturels de migration cellulaire peuvent également trouver des applications in vivo.

Le document US 2009/0093879 propose notamment un implant présentant en surface des motifs tridimensionnels micro- ou nanométriques. Ces motifs permettent notamment de contrôler l'adhésion de micro-organismes ou de fibroblastes à la surface de l'implant lorsque celui-ci est mis en place chez un être vivant, améliorant ainsi la cicatrisation des blessures. Ce document US 2009/0093879 suggère que les micro- ou nanostructures de surface peuvent guider les cellules à l'origine de la cicatrisation, leur permettant ainsi de s'organiser de manière ordonnée à la surface de l'implant.

Or un tel contrôle de la migration cellulaire selon une direction donnée pourrait également présenter des applications dans le domaine médical autres que l'organisation forcée des cellules autour d'un implant, telles que la migration dirigée des cellules à la surface d'une plaie ou la réalisation d'organes artificiels en ingénierie tissulaire.

Il existe donc un besoin pour de nouveaux dispositifs permettant de guider les cellules dans leur migration selon une direction choisie, dont l'efficacité ne dépend pas du type de cellule motile considérée, simples à mettre en oeuvre, peu invasifs pour les tissus et robuste dans le temps.

Par guider la migration cellulaire on entend, au sens de la présente demande, que l'on impose aux cellules de migrer préférentiellement dans une direction plutôt que dans toutes les autres. En d'autres termes, le guidage de la migration brise la symétrie de migration suivant la direction considérée. Le « guidage » de la migration diffère de « l'orientation » de la migration cellulaire où les cellules migrent préférentiellement dans deux directions opposées sans que l'une de ces directions ne soit favorisée par rapport à l'autre.

A cet effet, l'invention propose un dispositif de guidage de la migration cellulaire comprenant une surface support pourvue de cellules et un substrat, ledit substrat présentant une surface texturée placée en regard de la surface support et mise en contact avec les cellules placées sur la surface support de telle manière que les cellules soient confinées entre la surface support et la surface texturée, ladite surface texturée ayant une structure tridimensionnelle anisotrope présentant un motif de répétition selon un axe de répétition, ledit motif de répétition présentant une succession d'espaces de guidage adjacents selon l'axe de répétition, chacun desdits espaces de guidage étant adapté pour recevoir au moins une partie de l'une des cellules et orienté selon une direction d'anisotropie pour guider un déplacement des cellules dans la direction d'anisotropie.

Ainsi le dispositif de guidage selon l'invention peut assurer un contrôle de la migration des cellules par l'intermédiaire d'une structure particulière au sein de laquelle les cellules confinées sont reçues et déplacées dans une direction (un sens) privilégié, indépendamment du type de cellule utilisé. Par ailleurs, le dispositif de guidage est peu invasif car il repose sur l'application d'une surface texturée sur une surface support. Enfin, le dispositif de guidage peut être obtenu de manière simple, par une simple texturation de surface réalisable en grande série par moulage.

En outre, contrairement aux documents de l'art antérieur décrivant des canaux ou des micro-canaux contraignant s'alignement des cellules, la présente invention permet le guidage des cellules selon une direction d'anisotropie formant ainsi un réseau dans le plan compatible avec l'organisation d'un tissu selon une surface donnée.

Le dispositif de guidage selon l'invention peut notamment trouver des applications dans les domaines de la dermatologie, de l'implantologie ou de l'ingénierie tissulaire.

Par « structure anisotrope » ou « structure à géométrie anisotrope », on entend, au sens de la présente demande, une structure dont la géométrie possède une direction d'anisotropie déterminée selon un axe donné. La direction d'anisotropie de la structure anisotrope est notamment, dans le cadre de la présente invention, la direction préférentielle de la migration des cellules.

La surface texturée peut comprendre une surface de base et la structure tridimensionnelle anisotrope peut comprendre une pluralité de paires de surfaces de guidage, lesdites paires de surfaces de guidage étant adjacentes les unes aux autres selon l'axe de répétition et définissant le motif de répétition, chacune des paires comprenant des première et deuxième surfaces de guidage qui s'étendent depuis la surface de base en regard l'une de l'autre et qui délimitent entre elles l'un des espaces de guidage.

En particulier, la structure tridimensionnelle anisotrope peut comprendre une pluralité d'éléments de guidage en saillie par rapport à la surface de base, lesdits éléments de guidage étant adjacents les uns aux autres selon l'axe de répétition et portant chacun l'une des premières surfaces de guidage et l'une des deuxièmes surfaces de guidage, la première surface de guidage de l'un des éléments de guidage étant en regard de la deuxième de surface de guidage de l'élément de guidage adjacent.

Dans un mode de réalisation, les première et deuxième surfaces de guidage de chaque paire sont adaptées pour que la direction d'anisotropie s'étende selon l'axe de répétition. Dans chacune des paires de surfaces de guidage, la première surface de guidage peut alors être adaptée pour bloquer le déplacement des cellules dans une direction opposée à la deuxième surface de guidage et la deuxième surface de guidage peut être adaptée pour autoriser le déplacement des cellules dans une direction opposée à la première surface de guidage, de telle manière que la direction d'anisotropie soit orientée depuis la première surface vers la deuxième surface. Pour ce faire, la première surface de guidage peut être perpendiculaire à l'axe de répétition et la deuxième surface de guidage peut s'écarter de la première surface de guidage selon l'axe de répétition.

En particulier, dans une première variante, la deuxième surface de guidage peut être perpendiculaire à la surface de base et présenter une concavité tournée vers la première surface de guidage. Par exemple, la structure tridimensionnelle anisotrope peut comprendre une pluralité de rangées de projections triangulaires adjacentes selon l'axe de répétition, chacune desdites rangées comprenant au moins deux projections alignées selon un axe transversal perpendiculaire à l'axe de répétition, chacun des espaces de guidage comprenant une cavité sensiblement triangulaire avec une base formée sur la première surface de guidage de l'une des rangées de projections triangulaires et un sommet formé sur la deuxième surface de guidage de la rangée de projections triangulaires adjacente.

Dans une deuxième variante, la première surface de guidage peut être perpendiculaire à l'axe de répétition et la deuxième surface de guidage peut être inclinée par rapport à un plan perpendiculaire à la surface de base.

Dans un autre mode de réalisation, les première et deuxième surfaces de guidage de chaque paire sont adaptées pour que la direction d'anisotropie s'étende selon un axe transversal perpendiculaire à l'axe de répétition. Dans chacune des paires de surfaces de guidage, les première et deuxième surfaces de guidage peuvent alors être adaptées pour bloquer le déplacement des cellules dans l'une ou l'autre des directions de l'axe de répétition. En particulier, la structure tridimensionnelle anisotrope peut comprendre une pluralité de projections allongées adjacentes selon l'axe de répétition, chacune desdites projections allongées s'étendant selon l'axe transversal, chacun des espaces de guidage comprenant une rainure entre la première surface de guidage de l'une des projections allongées et la deuxième surface de guidage de la projection allongée adjacente.

Par ailleurs, l'espace de guidage peut présenter une dimension maximale, mesurée entre les première et deuxième surfaces de guidage, inférieure à 200 µm, de préférence inférieure à 100 µm, notamment correspondant sensiblement à une taille des cellules, par exemple comprise entre 5 µm et 60 µm, de préférence entre 15 µm et 30 µm. L'espace de guidage peut également présenter une profondeur inférieure à 200 µm, de préférence inférieure à 100 µm, notamment inférieure à une taille des cellules, par exemple inférieure à 6 µm.

Le substrat peut être non adhésif. Dans ce cas, il peut être constitué d'un matériau non adhésif tel qu'un polymère fluoré ou d'un matériau rendu non adhésif par traitement chimique tel que le greffage de molécules de polyéthylène glycol (PEG). La surface texturée non adhésive, c'est-à-dire sur laquelle les cellules ne peuvent pas adhérer, peut donc être retirée sans risque de détériorer les cellules.

En variante, le substrat peut être adhésif.

En outre, la surface support sur laquelle se déplacent les cellules peut être une surface artificielle telle qu'une surface de culture cellulaire (par exemple un gel), une lamelle de verre, l'intérieur d'un canal microfluidique, ou une surface de l'environnement naturel desdites cellules telle que la surface d'un tissu vivant ou la surface d'une plaie.

La surface support et la surface texturée peuvent être espacées d'une distance comprise entre 0 µm et 10 µm, de préférence entre 3 µm et 6 µm.

Au moins l'une des surfaces choisies parmi la surface support et la surface texturée peut comprendre au moins une saillie supplémentaire permettant de contrôler la distance entre la surface support et la surface texturée. En particulier, la saillie supplémentaire peut se présenter sous la forme d'un pilier de diamètre compris entre 100 µm et 500 µm, et de hauteur inférieure à 10 µm, de préférence entre 3 µm et 6 µm.

Selon des applications particulières, le dispositif de guidage peut se présenter sous la forme d'un pansement, d'un implant, d'une prothèse, d'un support de tissus artificiels, d'un canal microfluidique, d'un laboratoire sur puce intégrant des canaux, et de préférence, ledit dispositif de guidage est un pansement.

Selon un autre aspect, l'invention propose une méthode de guidage de la migration cellulaire mettant en oeuvre un dispositif de guidage tel que défini ci-dessus, ladite méthode de guidage prévoyant de mettre en contact les cellules placées sur la surface support avec la surface texturée du substrat de telle manière que les cellules soient confinées entre la surface support et la surface texturée, les cellules se déplaçant dans la direction d'anisotropie.

### Description des figures

D'autres objets et avantages de l'invention apparaîtront à la lecture de la description qui suit de modes de réalisation particuliers de l'invention donnés à titre d'exemple non limitatif, la description étant faite en référence aux dessins annexés dans lesquels :
- la figure 1 est une représentation en perspective d'un premier mode de réalisation d'un substrat d'un dispositif de guidage de migration cellulaire, le substrat présentant une surface texturée pourvue d'une pluralité de rangées de projections triangulaires définissant une succession de cavités de guidage triangulaires adjacents selon un axe de répétition, les cavités de guidages étant adaptées pour guider un déplacement de cellules portées par une surface support dans l'une des directions de l'axe de répétition,
- la figure 2 est une représentation en vue de dessus du substrat de la figure 1 dont la surface texturée est placée en regard d'une surface support dans un dispositif de guidage, la figure 2 illustrant le déplacement d'une cellule entre une surface support et la surface texturée du substrat,
- la figure 3 est une représentation en perspective du détail référencé III sur la figure 2, dans la direction de déplacement de la cellule,
- la figure 4A est une image en contraste de phase des cellules confinées sous les projections triangulaires, et la figure 4B est un histogramme donnant un biais induit dans la direction de migration des cellules,
- la figure 5 est une représentation en coupe du dispositif de guidage comprenant un substrat selon une variante du premier mode de réalisation de la figure 1, le substrat présentant une surface texturée pourvue d'une pluralité de projections allongées définissant une succession de rainures de guidage adjacentes selon l'axe de répétition, les rainures de guidages guidant le déplacement de cellules portées par la surface support dans l'une des directions de l'axe de répétition,
- la figure 6 est une représentation en perspective d'un deuxième mode de réalisation d'un substrat d'un dispositif de guidage de migration cellulaire, le substrat présentant une surface texturée pourvue d'une pluralité de projections allongées définissant une succession de rainures de guidage adjacentes selon un axe de répétition, les rainures de guidage étant adaptées pour guider un déplacement de cellules portées par une surface support dans l'une des directions d'un axe perpendiculaire à l'axe de répétition,
- la figure 7 est une représentation en vue de dessus du substrat de la figure 1 dont la surface texturée est placée en regard d'une surface support dans un dispositif de guidage, la figure 5 illustrant le déplacement d'une cellule entre une surface support et la surface texturée du substrat.

### Description détaillée

Sur les figures, les mêmes références désignent des éléments identiques ou analogues.

Les figures représentent un dispositif de guidage 1 de migration cellulaire comprenant une surface support 2 pourvue de cellules 3 et un substrat 5 adapté pour guider le déplacement des cellules selon un axe et dans l'une des deux directions de l'axe privilégiés.

Selon un mode préféré de réalisation, le substrat 5 est non adhésif, c'est-à-dire que les cellules ne peuvent pas adhérer sur le substrat 5, de manière, comme il apparaîtra de la suite de la description, à pouvoir retirer le substrat 5 de la surface support 2 sans détériorer les cellules. Un tel substrat 5 non adhésif est aussi appelé substrat anti-encrassement (ou « anti-fouling » selon la terminologie anglosaxone).

Le caractère non adhésif du substrat 5 correspond à une faible capacité d'adsorption protéique du substrat 5 ainsi qu'une faible capacité d'adhésion cellulaire, ce qui permet généralement de limiter les réactions inflammatoires.

Les matériaux non adhésifs pouvant convenir au substrat 5 non adhésif peuvent notamment être superhydrophobes - comme c'est le cas des polymères fluorés (par exemple, le polytétrafluoroéthylène (PTFE)) - ou des gels comme le polyacrylamide (PAM) ou le polyéthylène-glycol-diacrylate (PEGDA).

Alternativement, le substrat 5 non adhésif peut être constitué d'un matériau rendu non adhésif par traitement chimique.

Les traitements chimiques permettant de rendre le substrat 5 non adhésif peuvent notamment être le greffage sur le substrat d'une couche de gel mono-moléculaire, par exemple de type polyéthylène glycol (PEG), par exemple un PEG silanisé sur les oxydes ou thiolé sur les métaux ou conjugué avec un polyélectrolyte pour lui conférer la capacité de s'adsorber durablement par interaction électrostatique sur le substrat 5, comme c'est le cas pour le greffage de Polylysine-PEG (PLL-PEG).

De préférence, le matériau non adhésif est un polymère fluoré ou un matériau rendu non adhésif par traitement chimique tel que le greffage de molécules, par exemple de polyéthylène glycol (PEG).

Le substrat 5 peut, toutefois, notamment en fonction de l'application dans laquelle le dispositif de guidage 1 est mis en oeuvre, être adhésif.

Les matériaux adhésifs susceptibles de convenir pour le substrat adhésif 5 peuvent notamment être hydrophiles ou hydrophobes, en étant le cas échéant traités avec un promoteur d'adhésion cellulaire, et notamment choisis parmi :
- les plastiques biocompatibles : par exemple, le polystyrène (PS), couramment utilisé en culture cellulaire, des polymères siliconés comme le polydiméthylesiloxane (PDMS) notamment utilisés dans les laboratoires sur puce, des gels de copolymères blocks comme le styrène-éthylène/butylène-styrène (SEBS) utilisés pour la fabrication de pansements ou les polyacides lactiques et glycoliques (PLGA, PLA : hydrophile) qui sont biodégradables et peuvent être utilisés pour des implants ou comme support de tissus artificiels ; certains de ces plastiques peuvent avantageusement être activés par plasma oxygène pour augmenter leur hydrophilie et promouvoir l'adhésion cellulaire,
- les céramiques, généralement hydrophiles, telles que les oxydes métalliques ou les nitrures, comme par exemple le verre (SiO₂), le nitrure de silicium (Si₃N₄), le dioxyde de titane (TiO₂) ou d'autres ; ces matériaux sont utilisés en culture cellulaire, dans les laboratoires sur puce ou en implantologie ; ces matériaux peuvent avantageusement être activés par plasma oxygène pour augmenter leur hygrophylie et promouvoir l'adhésion cellulaire,
- les métaux inertes comme l'or, le platine, le palladium ou les métaux dont la surface oxydée ou nitrurée est stable comme le chrome ou le titane qui sont utilisés pour les implants ; avantageusement, les métaux peuvent être traités avec des molécules de la famille des thiols pour augmenter ou diminuer leur capacité d'adhésion cellulaire.

Il est, par ailleurs, possible de promouvoir l'adhésion cellulaire, en traitant chimiquement un matériau support. On peut alors utiliser :
- des polymères chargés (polyélectrolytes) qui s'adsorbent fortement par interaction électrostatique sur les surfaces oxydées (naturellement comme pour les oxydes ou artificiellement en activant les surfaces à l'aide d'un plasma oxygène) : par exemple la Poly-L-lysine (PLL) ou la Polyhornitine (PORN) ; ou
- des protéines d'adhésion cellulaire (Intégrines) ou de la matrice extracellulaire (Fibronectine, laminine, collagène) ou des peptides mimant ces protéines, comme le motif RGD (Arginyl glycyl aspartic acid).

Il est également possible, dans le cadre de la présente invention, de moduler l'adhésion du substrat 5 pour optimiser la motilité des cellules. En effet, le niveau d'adhésion des cellules sur un substrat 5 peut être modulé en traitant ce substrat avec un mélange ratiométrique de molécules adhésives et de molécules non-adhésives. Par exemple, un mélange de pLL-PEG et de pLL-PEG-RGD ou un mélange de pLL-PEG et de Fibronectine peuvent être utilisés.

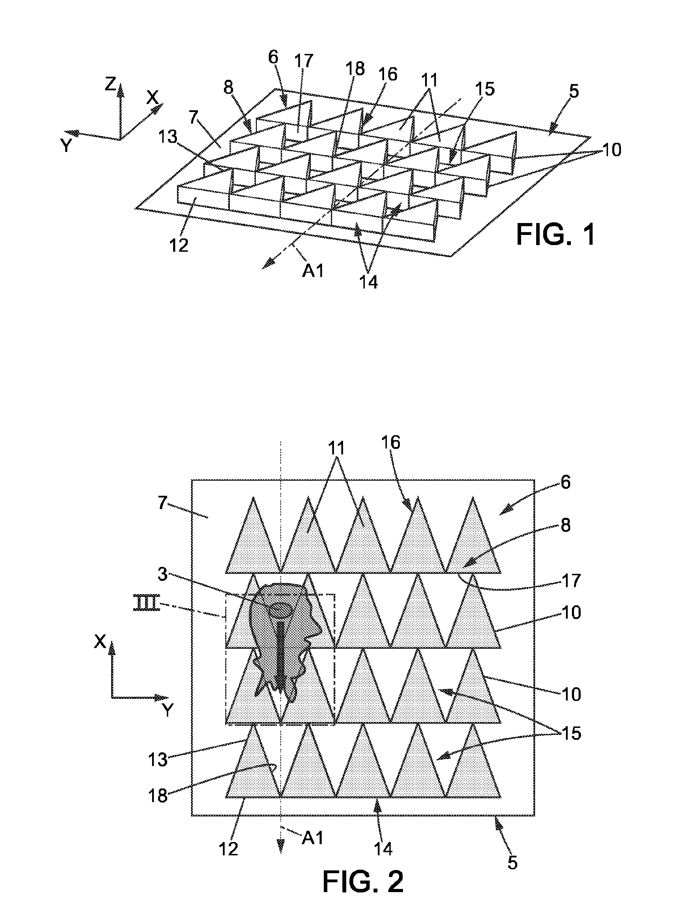

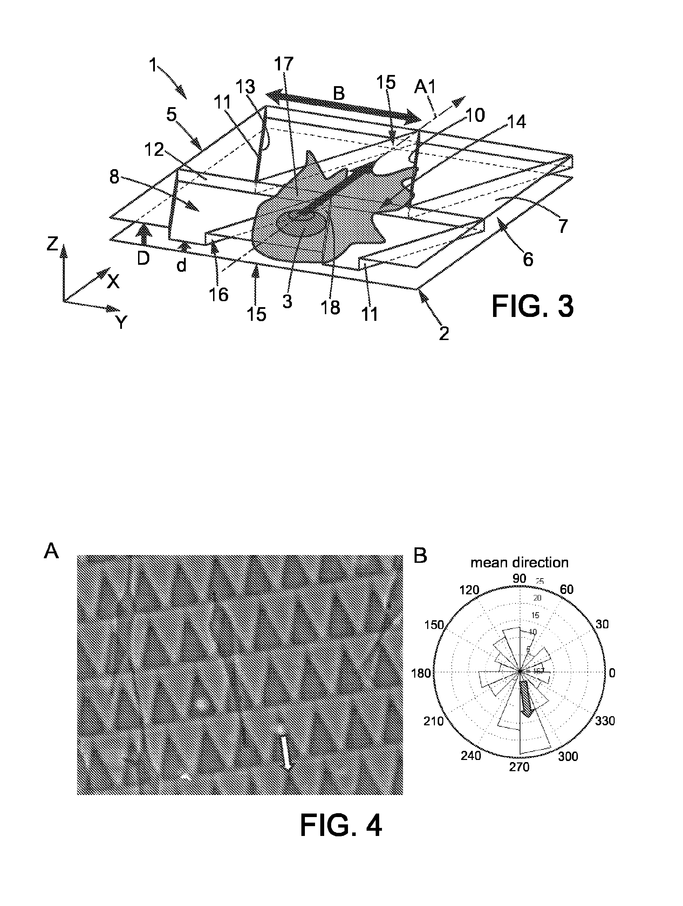

Le substrat 5 présente une surface texturée 6 ayant une surface de base 7 et une structure tridimensionnelle anisotrope 8, visible sur la figure 1 et représentée en transparence de la surface de base 7 sur les figures 2 et 3. La structure tridimensionnelle anisotrope 8 comprend une pluralité d'éléments de guidage en saillie par rapport à la surface de base 7 selon un axe vertical Z perpendiculaire à la surface de base 7. Les éléments de guidage sont agencés sur la surface de base 7 de manière adjacente les uns aux autres selon un axe de répétition X, perpendiculaire à l'axe vertical Z. Sur les figures, le substrat 5 est représenté à plat avec une description correspondante des axes pour clarifier l'orientation et le positionnement relatif des éléments du substrat 5. Comme il ressort de ce qui précède et comme il ressortira de la suite de la description, et notamment des applications du dispositif de guidage 1, le substrat 5 peut, toutefois, être déformable et présenter toute autre configuration que la configuration à plat représentée.

Dans le premier mode de réalisation représenté sur les figures 1 à 3, chaque élément de guidage se présente sous la forme d'une rangée 10 de projections triangulaires. Chaque rangée 10 comprend cinq projections triangulaires 11 alignées selon un axe transversal Y perpendiculaire à l'axe de répétition X et à l'axe vertical Z. Dans d'autres modes de réalisation, chaque rangée 10 peut comprendre deux, trois, quatre ou plus de cinq projections triangulaires 11.

Chaque projection triangulaire 11 présente une base 12 et un sommet 13. Dans chaque rangée 10, les bases 12 forment une première surface de guidage 14, perpendiculaire à la surface de base 7 et à l'axe de répétition X, et les sommets 13 forment une deuxième surface de guidage 16, perpendiculaire à la surface de base 7 et présentant une succession de concavités formant une denture.

La première surface de guidage 14 de l'une des rangées de projections triangulaires 10 est placée en regard de la deuxième de surface de guidage 16 de la rangée de projections triangulaires 10 adjacente. Les projections triangulaires 11 de deux rangées 10 adjacentes ont, par ailleurs, leurs bases 12 et leurs sommets 13 alignés. Les rangées de projections triangulaires 10 forment ainsi, entre elles, une pluralité de paires de surfaces de guidage adjacentes les unes aux autres selon l'axe de répétition X et définissent un motif de répétition selon cet axe de répétition X.

Chacune des paires de surfaces de guidage comprend l'une des premières surfaces de guidage 14 et la deuxième surface de guidage 16 en regard, en délimitant entre elles un espace de guidage. Dans le premier mode de réalisation, l'espace de guidage comprend une pluralité de cavités 15 sensiblement triangulaires avec chacune une base 17 formée sur la première surface de guidage 14 de l'une des rangées de projections triangulaires 10 et un sommet 18 formé sur la deuxième surface de guidage 16 de la rangée de projections triangulaires 10 adjacente. En particulier, pour chaque cavité 15, la base 17 est formée par des parties des bases 12 de deux projections triangulaires 11 de l'une des rangées 10 et le sommet 18 est formé par deux parois latérales convergeant l'une vers l'autre de deux projections triangulaires 11 de la rangée 10 adjacente. Le motif de répétition présente alors une succession de cavités 15 selon l'axe de répétition X.

Chacune des cavités 15 est adaptée pour recevoir au moins une partie de l'une des cellules 3. La cavité 15 présente une dimension maximale, mesurée entre les première 14 et deuxième 16 surfaces de guidage, inférieure à 200 µm, de préférence inférieure à 100 µm. Avantageusement, la dimension maximale de la cavité 15 correspond sensiblement à une taille des cellules 3 et est, par exemple, comprise entre 5 µm et 60 µm, de préférence entre 15 µm et 30 µm. La cavité 15 présente, par ailleurs, une profondeur, mesurée perpendiculairement à la surface de base 7, inférieure à 200 µm, de préférence inférieure à 100 µm. Avantageusement, la profondeur de la cavité 15 est inférieure à une taille des cellules 3, par exemple inférieure à 20 µm, ou encore inférieure à 6 µm. Par exemple, chacune des cavités 15 a une profondeur de 2 µm et est réalisée sous la forme d'un triangle équilatéral dont le côté B mesure 32 µm, soit une distance maximale entre les première 14 et deuxième 16 surfaces de guidage de 27,71 µm.

Dans le mode de réalisation représenté, les projections triangulaires 11 sont représentées attenantes les unes aux autres au sein d'une même rangée 10 et entre deux rangées 10 adjacentes. Des écarts entre les projections triangulaires 11 pourraient, toutefois, être prévus notamment pour adapter la dimension de la cavité 15. En outre, chaque rangée 10 a été représentée avec une pluralité de cavités 15, étant entendu que chaque rangée 10 pourrait ne comprendre qu'une seule cavité 15.

Une telle structure tridimensionnelle peut être réalisée, par exemple, par photolithographie, optionnellement suivie par une étape de gravure, ou par tout procédé de microfabrication.

En relation avec les figures 2 et 3, on décrit une méthode de guidage de migration cellulaire mettant en oeuvre le substrat 5 décrit ci-dessus.

La surface texturée 6 du substrat 5 est placée en regard de la surface support 2 et mise en contact avec les cellules 3 placées sur la surface support 2 de telle manière que les cellules 3 soient confinées entre la surface support 2 et la surface texturée 6. Le confinement des cellules 3 permet de renforcer leur guidage, en particulier lorsque le substrat 5 sur lequel les cellules 3 sont véhiculées est non adhésif.

La surface support 2 et la surface texturée 6 sont espacées d'une distance comprise entre 0 µm et 10 µm, de préférence entre 3 µm et 6 µm de sorte que l'épaisseur de la cellule 3 après confinement soit au moins comprise entre 3 et 6 µm pour permettre sa migration. La surface de base 7 du substrat 5 est placée à une distance D de la surface support 2, par exemple de 5 µm, et les projections triangulaires 11 sont placées à une distance d de la surface support 2, inférieure à la distance D entre la surface de base 7 et la surface support 2, par exemple de 3 µm.

La surface support 2 sur laquelle reposent les cellules 3 peut être une surface artificielle telle qu'une surface de culture cellulaire (par exemple un gel), une lamelle de verre, l'intérieur d'un canal microfluidique, ou une surface de l'environnement naturel desdites cellules telle que la surface d'un tissu vivant ou la surface d'une plaie.

Lorsque les cellules sont véhiculées sur un support portant la surface support 2 et présentant une rigidité supérieure à environ 20 kPa, il est souhaitable, dans le cadre de la présente invention, qu'au moins l'une des surfaces choisies parmi la surface support 2 et la surface texturée 6 comprenne au moins une saillie supplémentaire, non représentée, permettant de contrôler une distance entre la surface support 2 et la surface texturée 6 et ainsi d'éviter d'altérer les cellules 3. La hauteur de ces saillies est mesurée par rapport à la surface sur laquelle elles sont disposées. La saillie supplémentaire peut se présenter sous la forme d'un ou plusieurs piliers de diamètre compris entre 100 µm et 500 µm, et de hauteur inférieure à 10 µm, de préférence entre 3 µm et 6 µm, et en tout état de cause, de hauteur telle que l'épaisseur de la cellule 3 après confinement est au moins comprise entre 3 et 6 µm.

A l'inverse, lorsque les cellules sont véhiculées sur un support portant la surface support 2 et « mou », c'est-à-dire présentant une rigidité inférieure à environ 20 kPa, notamment comprise entre 100 Pa et 20 kPa, de préférence entre 500 Pa et 10 kPa, il n'est pas nécessaire qu'il possède des saillies supplémentaires dans la mesure où la surface support 2 est suffisamment « molle » pour permettre aux cellules 3 de ne pas être écrasées par le substrat, les cellules 3 définissant leur espace de confinement en déformant la surface support 2. Ces supports « mous » sont de type gel de faible rigidité ou tapis cellulaire. Les gels utilisés peuvent être des gels d'origine artificielle tels que le polyacrylamide (PAM) ou le polyéthylène-glycol-diacrylate (PEGDA) ou encore des gels d'origine naturelle comme le collagène, le matrigel ou l'acide hyaluronique (HA). La rigidité de ces gels peut être ajustée par leur composition et par les conditions de leur réticulation.

Chaque cavité 15 dont la deuxième surface de guidage 16 s'écarte de la première surface de guidage 14 selon l'axe de répétition X est orientée selon une direction d'anisotropie A1 parallèle à l'axe de répétition X et dirigée depuis la première surface de guidage 14 vers la deuxième surface 16.

Dans chaque cavité 15, la première surface de guidage 14 est adaptée pour bloquer le déplacement des cellules 3 dans une direction opposée à la deuxième surface de guidage 16 et la deuxième surface de guidage 16 est adaptée pour autoriser le déplacement des cellules 3 dans une direction opposée à la première surface de guidage 14.

Les cavités 15 peuvent alors guider un déplacement des cellules 3 portées par la surface support 2 dans la direction d'anisotropie A1, c'est-à-dire dans la direction de l'axe de répétition X allant de la base 17 vers le sommet 18 de la cavité 15.

La figure 4A est une image en contraste de phase des cellules confinées sous les projections triangulaires 10. Les triangles clairs sur la figure 4A représentent les cavités 15, les projections triangulaires 10 étant représentées par les triangles foncés. La figure 4B est un histogramme donnant un biais induit dans la direction de migration de 167 cellules confinées sous la structure décrite précédemment, après 24 heures de migration. Sur les figures 4A et 4B, une flèche donne la direction des pointes des cavités 15 triangulaires.

L'histogramme montre alors une direction privilégiée de migration correspondant à la direction selon laquelle les cavités 15 sont orientées.

La méthode de guidage qui vient d'être décrite utilise une surface texturée 6 dont la structure tridimensionnelle 8 ou texture est biaisée dans une direction, dite direction d'anisotropie A1, de manière à ce que les cellules 3 puissent appliquer une pression plus facilement dans une direction (ici, vers la deuxième surface de guidage 16) que dans une autre (ici, vers la première surface de guidage 14), ou puissent s'accrocher plus facilement dans une direction (ici, vers la première surface de guidage 14) que dans une autre (ici, vers la deuxième surface de guidage 16), ou puissent se déformer plus facilement dans une direction (ici, vers la deuxième surface de guidage 16) que dans une autre (ici, vers la première surface de guidage 14). Une telle anisotropie d'interaction entre la cellule 3 et le substrat 5 induit une direction de migration préférentielle, à l'image de la structure dite du cliquet Brownien (« Brownian ratchet ») décrite ci-dessus.

Dans le mode de réalisation décrit précédemment, la direction d'anisotropie A1 est définie par une concavité tournée vers la première surface de guidage 14 et formée par deux parois latérales rectilignes convergeant l'une vers l'autre. D'autres formes peuvent, toutefois, être prévues pour l'espace de guidage de manière à réaliser une concavité tournée vers la première surface de guidage 14 et, de là, une direction d'anisotropie A1 parallèle à l'axe de répétition X, allant de la première surface de guidage 14 à la deuxième surface de guidage 16. Par exemple, la concavité pourrait être arrondie, en étant notamment formée par deux parois latérales courbes convergeant l'une vers l'autre ou par une seule paroi latérale courbe.

L'invention n'est, par ailleurs, pas limitée à la réalisation d'une concavité pour orienter la direction d'anisotropie A1 parallèlement à l'axe de répétition X, depuis la première surface de guidage 14 vers la deuxième surface de guidage 16.

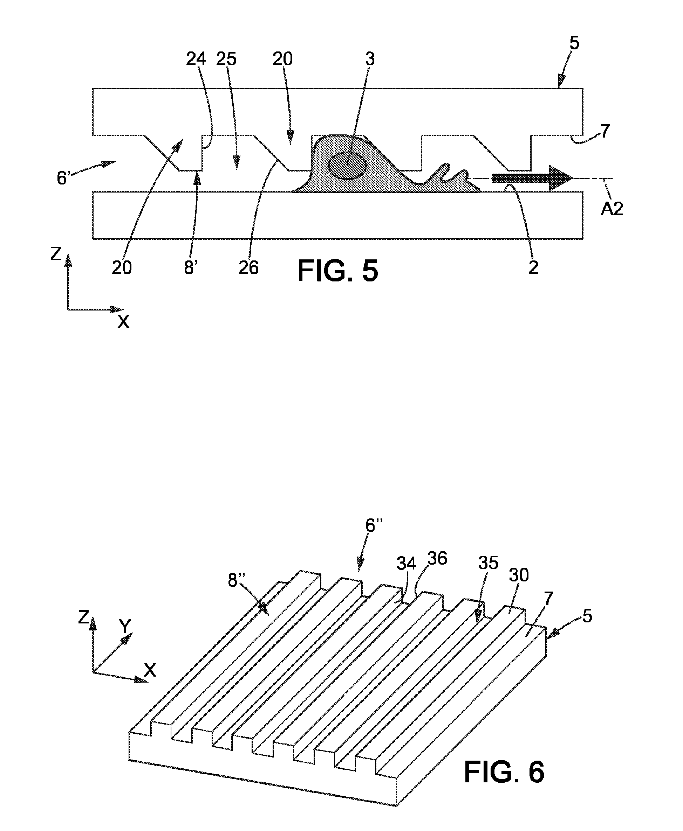

Par exemple, dans une variante représentée sur la figure 5, les éléments de guidage sont réalisés sous la forme de projections allongées 20 s'étendant chacune selon l'axe transversal Y, perpendiculaire à l'axe de répétition X. Chaque projection allongée 20 porte une première surface de guidage 24, perpendiculaire à l'axe de répétition X, et une deuxième surface de guidage 26, inclinée par rapport à un plan perpendiculaire à la surface de base 7.

Dans cette variante, seule la surface texturée 6' du substrat 5 diffère de celle décrite précédemment. La description détaillée du substrat 5 et des autres éléments identiques ne sera donc pas reprise et l'on se réfèrera à la description qui en a déjà été faite pour plus de détails.

Comme décrit précédemment, la première surface de guidage 24 de l'une des projections allongées 20 est placée en regard de la deuxième de surface de guidage 26 de la projection allongée 20 adjacente. Les projections allongées 20 forment ainsi, entre elles, une pluralité de paires de surfaces de guidage adjacentes les unes aux autres selon l'axe de répétition X et définissent un motif de répétition selon cet axe de répétition X.

Chacune des paires de surfaces de guidage comprend l'une des premières surfaces de guidage 24 et la deuxième surface de guidage 26 en regard, en délimitant entre elles un espace de guidage.

Dans cette variante du premier mode de réalisation, l'espace de guidage comprend une rainure 25 s'étendant selon l'axe transversale Y. Le motif de répétition présente alors une succession de rainures 25 selon l'axe de répétition X. Chacune des rainures 25 est adaptée pour recevoir au moins une partie de l'une des cellules 3. La rainure 25 présente une dimension maximale, mesurée au fond de la rainure 25 entre les première 24 et deuxième surfaces 26 de guidage, inférieure à 200 µm, de préférence inférieure à 100 µm. Avantageusement, la dimension maximale de la rainure 25 correspond sensiblement à une taille des cellules 3 et est, par exemple, comprise entre 5 µm et 60 µm, de préférence entre 15 µm et 30 µm. La rainure 25 présente, par ailleurs, une profondeur, mesurée perpendiculairement à la surface de base 7, inférieure à 200 µm, de préférence inférieure à 100 µm. Avantageusement, la profondeur de la rainure 25 est inférieure à une taille des cellules 3, par exemple inférieure à 20 µm, ou encore inférieure à 6 µm.

Une telle structure tridimensionnelle peut être réalisée, par exemple, par photolithographie, optionnellement suivie par une étape de gravure anisotrope type RIE, ICP ou DRIE en inclinant l'échantillon lors de la photolithographie ou gravure pour obtenir les faces inclinées, ou encore par photolithographie à niveau de gris (« Gray-tone lithography »).

Comme représenté sur la figure 5, chaque rainure 25 dont la deuxième surface de guidage 26 s'écarte de la première surface de guidage 24 selon l'axe de répétition X est orientée selon une direction d'anisotropie A2 parallèle à l'axe de répétition X et dirigée depuis la première surface de guidage 24 vers la deuxième surface de guidage 26. Dans chaque rainure 25, la première surface de guidage 24 bloque le déplacement des cellules 3 dans une direction opposée à la deuxième surface de guidage 26 et la deuxième surface de guidage 26 autorise le déplacement des cellules 3 dans une direction opposée à la première surface de guidage 24. Les rainures 25 peuvent alors guider un déplacement des cellules 3 portées par la surface support 2 dans la direction d'anisotropie A2, c'est-à-dire dans la direction de l'axe de répétition X allant de la première surface de guidage 24 vers la deuxième surface de guidage 26.

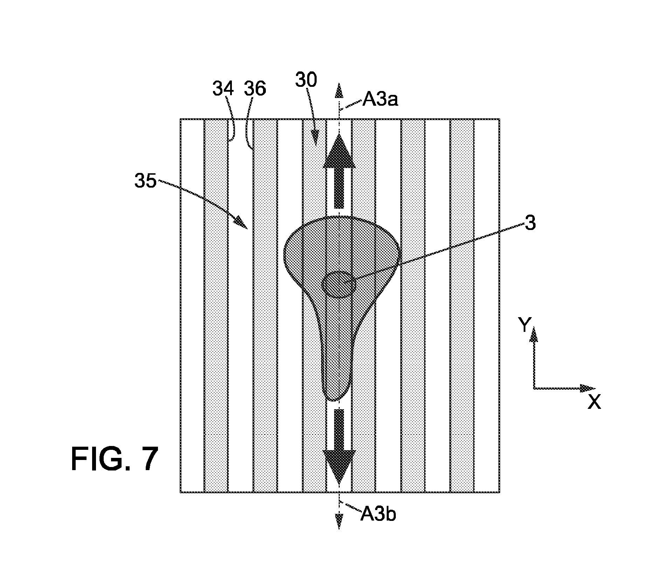

Les figures 6 et 7 représentent un deuxième mode de réalisation de l'invention dans lequel les espaces de guidage 35 sont orientés dans une direction d'anisotropie A3a, A3b qui s'étend selon l'axe transversal Y, perpendiculaire à l'axe de répétition X, de manière à guider le déplacement des cellules 3 selon l'axe transversal Y.

Dans ce deuxième mode de réalisation, seule la surface texturée 6" du substrat 5 diffère de celle du premier mode de réalisation décrit précédemment. La description détaillée du substrat 5 et des autres éléments identiques ne sera donc pas reprise et l'on se réfèrera à la description qui en est faite en relation avec le premier mode de réalisation pour plus de détails.

Les éléments de guidage sont réalisés sous la forme de projections allongées 30 s'étendant chacune selon l'axe transversal Y. Chaque projection allongée 30 porte des première 34 et deuxième 36 surfaces de guidage, perpendiculaires à l'axe de répétition X.

Comme décrit précédemment, la première surface de guidage 34 de l'une des projections allongées 30 est placée en regard de la deuxième de surface de guidage 36 de la projection allongée 30 adjacente. Les projections allongées 30 forment ainsi, entre elles, une pluralité de paires de surfaces de guidage adjacentes les unes aux autres selon l'axe de répétition X et définissent un motif de répétition selon cet axe de répétition X.

Chacune des paires de surfaces de guidage comprend l'une des premières surfaces de guidage 34 et la deuxième surface de guidage 36 en regard, en délimitant entre elles un espace de guidage.

Dans ce deuxième mode de réalisation, l'espace de guidage comprend une rainure 35 qui s'étend selon l'axe transversal Y. Le motif de répétition présente alors une succession de rainures 35 selon l'axe de répétition X. Chacune des rainures 35 est adaptée pour recevoir au moins une partie de l'une des cellules 3. La rainure 35 présente une dimension maximale, mesurée entre les première 34 et deuxième 36 surfaces de guidage, inférieure à 200 µm, de préférence inférieure à 100 µm. Avantageusement, la dimension maximale de la rainure 35 correspond sensiblement à une taille des cellules 3 et est, par exemple, comprise entre 5 µm et 60 µm, de préférence entre 15 µm et 30 µm. La rainure 35 présente, par ailleurs, une profondeur, mesurée perpendiculairement à la surface de base 7, inférieure à 200 µm, de préférence inférieure à 100 µm. Avantageusement, la profondeur de la rainure 35 est inférieure à une taille des cellules 3, par exemple inférieure à 20 µm, ou encore inférieure à 6 µm.

Chaque rainure 35 dont les première 34 et deuxième 36 surfaces de guidage s'étendent selon l'axe transversal Y peut être orientée selon l'une des directions d'anisotropie A3a, A3b parallèle à l'axe transversal Y par tout moyen approprié, comme par exemple des crans ou cliquets agencées sur les première 34 et deuxième 36 surfaces de guidage ou sur le fond de la rainure 35. Dans chaque rainure 35, les première 34 et deuxième 36 surfaces de guidage bloquent le déplacement des cellules 3 dans l'une ou l'autre des directions de l'axe de répétition X. Les rainures 35 peuvent alors guider un déplacement des cellules 3 portées par la surface support 2 dans l'une des directions d'anisotropie A3a, A3b.

Le dispositif de guidage 1 selon l'invention peut notamment trouver de nombreuses applications de guidage de la migration des cellules *in vivo* ou *in vitro.*

On entend par guidage *in vitro* le guidage de la migration des cellules en culture dans un milieu entièrement artificiel. Les cellules peuvent, par exemple, être cultivées sur une surface support artificielle telle qu'une boite de culture cellulaire, la surface texturée étant alors appliquée sur la surface support pour confiner les cellules. Dans un autre mode de réalisation, la surface texturée peut être intégrée sur l'une des faces d'un canal microfluidique pour guider la migration des cellules dans ledit canal microfluidique. Les surfaces texturées du dispositif selon l'invention peuvent être utilisées, soit pour l'étude des mécanismes biologiques et physiques de migration et de prolifération des cellules en culture, soit pour réaliser du tri cellulaire en séparant les cellules suivant leurs caractéristiques de migration. Alternativement, le dispositif selon l'invention peut permettre de guider des cellules sur des supports bidimensionnels ou tridimensionnels couvert au moins partiellement d'une surface de guidage texturée pour la réalisation d'organes artificiels (ingénierie tissulaire). Le dispositif selon l'invention peut trouver une application dans tout domaine nécessitant de guider les cellules artificiellement et indépendamment de leur comportement chimiotactique.

On entend par guidage *in vivo* le guidage de la prolifération et de la migration des cellules chez un être vivant, par exemple chez l'homme. Dans ce cas, la surface support est constituée du support physiologique naturel des cellules sur lequel est appliquée la surface texturée. Selon un mode préféré de réalisation de guidage *in vivo*, la surface texturée peut être utilisée pour guider les cellules présentes à la surface d'une plaie de manière à favoriser la répartition des cellules sur la plaie. Le dispositif est alors un pansement présentant des microstructures à sa surface. Selon un autre mode de réalisation de guidage *in vivo,* la surface texturée peut être utilisée pour guider les cellules autour d'une prothèse de manière à favoriser la répartition des cellules autour de la prothèse. Selon encore un autre mode de réalisation de guidage *in vivo,* la surface texturée peut être utilisée pour guider les cellules autour d'un film ou d'un pansement interne placé à l'intérieur du corps d'un être vivant de manière à favoriser la répartition des cellules dans ou autour d'un organe.

Dans une application particulière, le dispositif de guidage peut avantageusement être mis en oeuvre *in vivo*, comme pansement, pour favoriser la cicatrisation d'une plaie. Outre l'application particulièrement avantageuse comme pansement, le dispositif de guidage selon l'invention trouve des applications comme implant, prothèse, support de tissus artificiels, canal microfluidique, ou laboratoire sur puce intégrant des canaux.

## Revendications

1. Dispositif de guidage (1) de la migration cellulaire comprenant une surface support (2) pourvue de cellules (3), et un substrat (5), ledit substrat (5) présentant une surface texturée (6 ; 6' ; 6") placée en regard de la surface support (2) et mise en contact avec les cellules (3) placées sur la surface support (2) de telle manière que les cellules (3) soient confinées entre la surface support (2) et la surface texturée (6 ; 6' ; 6"), ladite surface texturée (6 ; 6' ; 6") ayant une structure tridimensionnelle anisotrope (8 ; 8' ; 8") présentant un motif de répétition selon un axe de répétition (X), ledit motif de répétition présentant une succession d'espaces de guidage (15 ; 25 ; 35) adjacents selon l'axe de répétition (X), chacun desdits espaces de guidage (15 ; 25 ; 35) étant adapté pour recevoir au moins une partie de l'une des cellules (3) et orienté selon une direction d'anisotropie (A1 ; A2 ; A3a, A3b) pour guider un déplacement des cellules (3) dans la direction d'anisotropie (A1 ; A2 ; A3a, A3b).

2. Dispositif de guidage (1) selon la revendication 1, dans lequel la surface texturée (6 ; 6' ; 6") comprend une surface de base (7) et la structure tridimensionnelle anisotrope (8 ; 8' ; 8") comprend une pluralité de paires de surfaces de guidage, lesdites paires de surfaces de guidage étant adjacentes les unes aux autres selon l'axe de répétition (X) et définissant le motif de répétition, chacune des paires comprenant des première (14 ; 24 ; 34) et deuxième (16 ; 26 ; 36) surfaces de guidage qui s'étendent depuis la surface de base (7) en regard l'une de l'autre et qui délimitent entre elles l'un des espaces de guidage (15 ; 25 ; 35).

3. Dispositif de guidage (1) selon la revendication 2, dans lequel la structure tridimensionnelle anisotrope (8 ; 8' ; 8") comprend une pluralité d'éléments de guidage (10 ; 20 ; 30) en saillie par rapport à la surface de base (7), lesdits éléments de guidage (10 ; 20 ; 30) étant adjacents les uns aux autres selon l'axe de répétition (X) et portant chacun l'une des premières surfaces de guidage (14 ; 24 ; 34) et l'une des deuxièmes surfaces de guidage (16 ; 26 ; 36), la première surface de guidage (14 ; 24 ; 34) de l'un des éléments de guidage (10 ; 20 ; 30) étant en regard de la deuxième de surface de guidage (16 ; 26 ; 36) de l'élément de guidage (10 ; 20 ; 30) adjacent.

4. Dispositif de guidage (1) selon la revendication 2 ou 3, dans lequel les première (14 ; 24) et deuxième surfaces de guidage (16 ; 26) de chaque paire sont adaptées pour que la direction d'anisotropie (A1 ; A2) s'étende selon l'axe de répétition (X).

5. Dispositif de guidage (1) selon la revendication 4, dans lequel, dans chacune des paires de surfaces de guidage, la première surface de guidage (14 ; 24) est adaptée pour bloquer le déplacement des cellules (3) dans une direction opposée à la deuxième surface de guidage (16 ; 26) et la deuxième surface de guidage (16 ; 26) est adaptée pour autoriser le déplacement des cellules (3) dans une direction opposée à la première surface de guidage (14 ; 24), de telle manière que la direction d'anisotropie (A1 ; A2) soit orientée depuis la première surface de guidage (14 ; 24) vers la deuxième surface de guidage (16 ; 26).

6. Dispositif de guidage (1) selon la revendication 5, dans lequel la première surface de guidage (14 ; 24) est perpendiculaire à l'axe de répétition (X) et la deuxième surface de guidage (16 ; 26) s'écarte de la première surface de guidage (14 ; 24) selon l'axe de répétition (X).

7. Dispositif de guidage (1) selon la revendication 6, dans lequel la deuxième surface de guidage (16) est perpendiculaire à la surface de base (7) et présente une concavité tournée vers la première surface de guidage (14).

8. Dispositif de guidage (1) selon la revendication 7, dans lequel la structure tridimensionnelle anisotrope (8) comprend une pluralité de rangées de projections triangulaires (10) adjacentes selon l'axe de répétition (X), chacune desdites rangées (10) comprenant au moins deux projections (11) alignées selon un axe transversal (Y) perpendiculaire à l'axe de répétition (X), chacun des espaces de guidage comprenant une cavité (15) sensiblement triangulaire avec une base (17) formée sur la première surface de guidage (14) de l'une des rangées de projections triangulaires (10) et un sommet (18) formé sur la deuxième surface de guidage (16) de la rangée de projections triangulaires (10) adjacente.

9. Dispositif de guidage (1) selon la revendication 6, dans lequel la première surface de guidage (24) est perpendiculaire à l'axe de répétition et la deuxième surface de guidage (26) est inclinée par rapport à un plan perpendiculaire à la surface de base (7).

10. Dispositif de guidage (1) selon la revendication 2 ou 3, dans lequel les première (34) et deuxième (36) surfaces de guidage de chaque paire sont adaptées pour que la direction d'anisotropie (A3a, A3b) s'étende selon un axe transversal (Y) perpendiculaire à l'axe de répétition (X).

11. Dispositif de guidage (1) selon la revendication 10, dans lequel, dans chacune des paires de surfaces de guidage, les première (34) et deuxième (36) surfaces de guidage sont adaptées pour bloquer le déplacement des cellules (3) dans l'une ou l'autre des directions de l'axe de répétition (X).

12. Dispositif de guidage (1) selon la revendication 11, dans lequel la structure tridimensionnelle anisotrope (8") comprend une pluralité de projections allongées (30) adjacentes selon l'axe de répétition (X), chacune desdites projections allongées (30) s'étendant selon l'axe transversal (Y), chacun des espaces de guidage comprenant une rainure (35) entre la première surface de guidage (34) de l'une des projections allongées (30) et la deuxième surface de guidage (36) de la projection allongée (30) adjacente.

13. Dispositif de guidage (1) selon l'une quelconque des revendications 2 à 12, dans lequel l'espace de guidage (15 ; 25 ; 35) présente une dimension maximale, mesurée entre les première (14 ; 24 ; 34) et deuxième surfaces de guidage (16 ; 26 ; 36), inférieure à 200 µm, de préférence inférieure à 100 µm, notamment correspondant sensiblement à une taille des cellules (3), par exemple comprise entre 5 µm et 60 µm, de préférence entre 15 µm et 30 µm.

14. Dispositif de guidage (1) selon l'une quelconque des revendications 2 à 13, dans lequel l'espace de guidage (15 ; 25 ; 35) présente une profondeur inférieure à 200 µm, de préférence inférieure à 100 µm, notamment inférieure à une taille des cellules (3), par exemple inférieure à 6 µm.

15. Dispositif de guidage (1) selon l'une quelconque des revendications 1 à 14, dans lequel le substrat (5) est non adhésif.

16. Dispositif de guidage (1) selon la revendication 15, dans lequel le substrat (5) non adhésif est constitué d'un matériau non adhésif tel qu'un polymère fluoré ou d'un matériau rendu non adhésif par traitement chimique tel que le greffage de molécules de polyéthylène glycol (PEG).

17. Dispositif de guidage (1) selon l'une quelconque des revendications 1 à 14, dans lequel le substrat (5) est adhésif.

18. Dispositif de guidage (1) selon l'une quelconque des revendications 1 à 17, dans lequel la surface support (2) sur laquelle se déplacent les cellules (3) est une surface artificielle telle qu'une surface de culture cellulaire (par exemple un gel), une lamelle de verre, l'intérieur d'un canal microfluidique, ou une surface de l'environnement naturel desdites cellules telle que la surface d'un tissu vivant ou la surface d'une plaie.

19. Dispositif de guidage (1) selon l'une quelconque des revendications 1 à 18, dans lequel la surface support (2) et la surface texturée (6 ; 6' ; 6") sont espacées d'une distance comprise entre 0 µm et 10 µm, de préférence entre 3 µm et 6 µm.

20. Dispositif de guidage (1) selon la revendication 19, dans lequel au moins l'une des surfaces choisies parmi la surface support (2) et la surface texturée (6 ; 6' ; 6") comprend au moins une saillie supplémentaire permettant de contrôler la distance entre la surface support (2) et la surface texturée (6 ; 6' ; 6").

21. Dispositif de guidage (1) selon la revendication 20, dans lequel la saillie supplémentaire se présente sous la forme d'un pilier de diamètre compris entre 100 µm et 500 µm, et de hauteur inférieure à 10 µm, de préférence entre 3 µm et 6 µm.

22. Dispositif de guidage (1) selon l'une quelconque des revendications 1 à 21, se présentant sous la forme d'un pansement, d'un implant, d'une prothèse, d'un support de tissus artificiels, d'un canal microfluidique, d'un laboratoire sur puce intégrant des canaux, et de préférence, ledit dispositif de guidage (1) est un pansement.

23. Méthode de guidage de la migration cellulaire mettant en oeuvre un dispositif de guidage (1) selon l'une quelconque des revendications 1 à 22, ladite méthode de guidage prévoyant de mettre en contact les cellules (3) placées sur la surface support (2) avec la surface texturée (6 ; 6' ; 6") du substrat (5) de telle manière que les cellules (3) soient confinées entre la surface support (2) et la surface texturée (6 ; 6' ; 6"), les cellules (3) se déplaçant dans la direction d'anisotropie (A1 ; A2 ; A3a, A3b).

## Patentansprüche

1. Vorrichtung zur Führung (1) der Zellmigration, umfassend eine mit Zellen (3) versehene Trägeroberfläche (2) und ein Substrat (5), wobei das Substrat (5) eine texturierte Oberfläche (6; 6'; 6") aufweist, die der Trägeroberfläche (2) gegenüber platziert ist und mit den Zellen (3), die auf der Trägeroberfläche (2) platziert sind, so in Kontakt gebracht wird, dass die Zellen (3) auf den Raum zwischen der Trägeroberfläche (2) und der texturierten Oberfläche (6; 6'; 6") beschränkt sind, wobei die texturierte Oberfläche (6; 6'; 6") eine dreidimensionale anisotrope Struktur (8; 8'; 8") hat, die ein Wiederholungsmotiv an einer Wiederholungsachse (X) aufweist, wobei das Wiederholungsmotiv eine Folge von aneinandergrenzenden Führungsräumen (15; 25; 35) entlang der Wiederholungsachse (X) darstellt, wobei jeder der Führungsräume (15; 25; 35) so ausgestaltet ist, dass er mindestens einen Teil einer der Zellen (3) aufnimmt und in einer Anisotropie-Richtung (A1; A2; A3a; A3b) ausgerichtet ist, um ein Wandern der Zellen (3) in der Richtung der Anisotropie (A1; A2; A3a; A3b) zu leiten.

2. Vorrichtung zur Führung (1) nach Anspruch 1, wobei die texturierte Oberfläche (6; 6'; 6") eine Basisoberfläche (7) umfasst und die dreidimensionale anisotrope Struktur (8; 8'; 8") eine Vielzahl von Führungsoberflächenpaaren umfasst, wobei die Führungsoberflächenpaare entlang der Wiederholungsachse (X) aneinander angrenzen und das Wiederholungsmotiv definieren, wobei jedes Paar erste (14; 24; 34) und zweite (16; 26; 36) Führungsoberflächen umfasst, die sich einander gegenüber von der Basisoberfläche (7) aus erstrecken und die jeweils zusammen einen der Führungsräume (15; 25; 35) begrenzen.

3. Vorrichtung zur Führung (1) nach Anspruch 2, wobei die dreidimensionale anisotrope Struktur (8; 8'; 8") eine Vielzahl von im Verhältnis zur Basisoberfläche (7) vorspringenden Führungselementen (10; 20; 30) umfasst, wobei die Führungselemente (10; 20; 30) entlang der Wiederholungsachse (X) aneinander angrenzen und jedes Führungselement eine der ersten Führungsoberflächen (14; 24; 34) und eine der zweiten Führungsoberflächen (16; 26; 36) trägt, wobei die erste Führungsoberfläche (14; 24; 34) eines der Führungselemente (10; 20; 30) der zweiten Führungsoberfläche (16; 26; 36) des angrenzenden Führungselements (10; 20; 30) gegenüberliegt.

4. Vorrichtung zur Führung (1) nach Anspruch 2 oder 3, wobei die ersten Führungsoberflächen (14; 24) und die zweiten Führungsoberflächen (16; 26) jeden Paares so ausgestaltet sind, dass sich die Richtung der Anisotropie (A1; A2) entlang der Wiederholungsachse (X) erstreckt.

5. Vorrichtung zur Führung (1) nach Anspruch 4, wobei in jedem der Führungsoberflächenpaare die erste Führungsoberfläche (14; 24) so ausgestaltet ist, dass sie die Wanderung der Zellen (3) in einer der zweiten Führungsoberfläche (16; 26) entgegengesetzten Richtung blockieren und die zweite Führungsoberfläche (16; 26) so ausgestaltet ist, dass sie die Wanderung der Zellen (3) in einer der ersten Führungsoberfläche (14; 24) entgegengesetzten Richtung erlaubt, so dass die Richtung der Anisotropie (A1; A2) von der ersten Führungsoberfläche (14; 24) zur zweiten Führungsoberfläche (16; 26) hin orientiert ist.

6. Vorrichtung zur Führung (1) nach Anspruch 5, wobei die erste Führungsoberfläche (14; 24) zur Wiederholungsachse (X) senkrecht ist und die zweite Führungsoberfläche (16; 26) sich entlang der Wiederholungsachse (X) von der ersten Führungsoberfläche (14; 24) weg erstreckt.

7. Vorrichtung zur Führung (1) nach Anspruch 6, wobei die zweite Führungsoberfläche (16) zur Basisoberfläche (7) senkrecht ist und eine zur ersten Führungsoberfläche (14) gewandte Vertiefung aufweist.

8. Vorrichtung zur Führung (1) nach Anspruch 7, wobei die dreidimensionale anisotrope Struktur (8) eine Vielzahl von entlang der Wiederholungsachse (X) aneinandergrenzenden Reihen von dreieckförmigen Vorsprüngen (10) umfasst, wobei jede Reihe (10) mindestens zwei Vorsprünge (11) umfasst, die entlang einer transversalen Achse (Y), die zur Wiederholungsachse (X) senkrecht ist, angeordnet sind, wobei jeder der Führungsräume eine im Wesentlichen dreieckförmige Aushöhlung (15) mit einer Basis (17) umfasst, die auf der ersten Führungsoberfläche (14) einer der Reihen der dreieckförmigen Vorsprünge (10) gebildet ist, und einen Scheitelpunkt (18) umfasst, der auf der zweiten Führungsoberfläche (16) der Reihe der aneinandergrenzenden dreieckförmigen Vorsprünge (10) gebildet ist.

9. Vorrichtung zur Führung (1) nach Anspruch 6, wobei die erste Führungsoberfläche (24) senkrecht zur Wiederholungsachse ist und die zweite Führungsoberfläche (26) im Verhältnis zu einer Ebene geneigt ist, die zu der Basisoberfläche (7) senkrecht ist.

10. Vorrichtung zur Führung (1) nach Anspruch 2 oder 3, wobei die ersten Führungsoberflächen (34) und die zweiten Führungsoberflächen (36) jeden Paares so ausgestaltet sind, dass sich die Richtung der Anisotropie (A3a, A3b) sich entlang einer transversalen Achse (Y) erstreckt, die zur Wiederholungsachse (X) senkrecht ist.

11. Vorrichtung zur Führung (1) nach Anspruch 10, wobei in jedem Führungsoberflächenpaar, die ersten (34) und die zweiten Führungsoberflächen (36) so ausgestaltet sind, dass sie die Wanderung der Zellen (3) in die eine oder die andere der Richtungen der Wiederholungsachse (X) blockieren.

12. Vorrichtung zur Führung (1) nach Anspruch 11, wobei die dreidimensionale anisotrope Struktur (8") eine Vielzahl von länglichen Vorsprüngen (30) umfasst, die entlang der Wiederholungsachse (X) aneinander angrenzenden, wobei sich jeder der länglichen Vorsprünge (30) entlang der transversalen Achse (Y) erstreckt, wobei jeder Führungsraum eine Rille (35) zwischen der ersten Führungsoberfläche (34) einer der länglichen Vorsprünge (30) und der zweiten Führungsoberfläche (36) des angrenzenden Vorsprungs (30) umfasst.

13. Vorrichtung zur Führung (1) nach einem der Ansprüche 2 bis 12, wobei der Führungsraum (15; 25; 35) eine maximale, zwischen den ersten (14; 24; 34) und den zweiten Führungsoberflächen (16; 26; 36) gemessene Abmessung unter 200 µm, bevorzugt unter 100 µm darstellt, die insbesondere im Wesentlichen einer Größe der Zellen (3) entspricht, zum Beispiel zwischen 5 µm und 60 µm, bevorzugt zwischen 15 µm und 30 µm.

14. Vorrichtung zur Führung (1) nach einem der Ansprüche 2 bis 13, wobei der Führungsraum (15; 25; 35) eine Tiefe von weniger als 200 µm, bevorzugt von weniger als 100 µm, insbesondere geringer als eine Größe der Zellen (3), zum Beispiel weniger als 6 µm, aufweist.

15. Vorrichtung zur Führung (1) nach einem der Ansprüche 1 bis 14, wobei das Substrat (5) nicht-haftend ist.

16. Vorrichtung zur Führung (1) nach Anspruch 15, wobei das nicht haftende Substrat (5) aus einem nicht-haftenden Material wie einem Fluorpolymer oder einem Material, das durch chemische Behandlung, wie die Übertragung von Polyethylenglycol (PEG)-Molekülen, nicht-haftend gemacht wurde, besteht.

17. Vorrichtung zur Führung (1) nach einem der Ansprüche 1 bis 14, wobei das Substrat (5) haftend ist.

18. Vorrichtung zur Führung (1) nach einem der Ansprüche 1 bis 17, wobei die Trägeroberfläche (2), auf der die Zellen (3) wandern, eine künstliche Oberfläche ist, wie zum Beispiel eine Zellkulturoberfläche (zum Beispiel ein Gel), ein Objektträger aus Glas, das Innere eines Mikrofluidkanals oder eine Oberfläche aus der natürlichen Umgebung der Zellen, wie ein lebendes Gewebe oder die Oberfläche einer Wunde.

19. Vorrichtung zur Führung (1) nach einem der Ansprüche 1 bis 18, wobei die Trägeroberfläche (2) und die texturierte Oberfläche (6; 6'; 6") im Abstand von zwischen 0 µm und 10 µm, bevorzugt zwischen 3 µm und 6 µm voneinander entfernt angeordnet sind.

20. Vorrichtung zur Führung (1) nach Anspruch 19, wobei mindestens eine der aus der Trägeroberfläche (2) und der texturierten Oberfläche (6; 6'; 6") ausgewählte Oberfläche mindestens einen zusätzlichen Vorsprung umfasst, der es ermöglicht, den Abstand zwischen der Trägeroberfläche (2) und der texturierten Oberfläche (6; 6'; 6") zu kontrollieren.

21. Vorrichtung zur Führung (1) nach Anspruch 20, wobei der zusätzliche Vorsprung die Form einer Stütze mit einem Durchmesser von zwischen 100 µm und 500 µm und einer Höhe von weniger als 10 µm, bevorzugt zwischen 3 µm und 6 µm aufweist.

22. Vorrichtung zur Führung (1) nach einem der Ansprüche 1 bis 21, wobei die Vorrichtung in Form eines Verbandes, eines Implantats, einer Prothese, eines Trägers künstlicher Gewebe, eines Mikrofluidkanals, eines Lab-on-Chip-Systems, das Kanäle einschließt, vorliegt, und die Vorrichtung zur Führung (1) bevorzugt ein Verband ist.

23. Verfahren zur Führung der Zellmigration, das eine Vorrichtung zur Führung (1) nach einem der Ansprüche 1 bis 22 verwendet, wobei das Führungsverfahren vorsieht, die Zellen (3), die auf der Trägeroberfläche (2) platziert sind, mit der texturierten Oberfläche (6; 6'; 6") des Substrats (5) in Kontakt zu bringen, so dass die Zellen (3) auf den Raum zwischen der Trägeroberfläche (2) und der texturierten Oberfläche (6; 6'; 6") beschränkt sind, wobei die Zellen in der Richtung der Anisotropie (A1; A2; A3a; A3b) wandern.

## Claims

1. Device (1) for guiding cell migration comprising a support surface (2) provided with cells (3) and a substrate (5), said substrate (5) having a textured surface (6; 6'; 6") disposed facing the support surface (2) and placed in contact with the cells (3) disposed on the support surface (2) so that the cells (3) are confined between the support surface (2) and the textured surface (6; 6'; 6"), said textured surface (6; 6'; 6") having an anisotropic three-dimensional structure (8; 8'; 8") having a repeating pattern along a repeat axis (X), said repeating pattern having a succession of adjacent guide spaces (15; 25; 35) along the repeat axis (X), each of said guide spaces (15; 25; 35) being configured to receive at least a part of one of the cells (3) and being oriented in a direction of anisotropy (A1; A2; A3a, A3b) in order to guide a movement of the cells (3) in the direction of anisotropy (A1; A2; A3a, A3b).

2. Guiding device (1) as claimed in claim 1, wherein the textured surface (6; 6'; 6") comprises a base surface (7) and the anisotropic three-dimensional structure (8; 8'; 8") comprises a plurality of pairs of guide surfaces, said pairs of guide surfaces being adjacent to one another along the repeat axis (X) and defining the repeating pattern, each of the pairs comprising first (14; 24; 34) and second (16; 26; 36) guide surfaces extending from the base surface (7) facing one another and bounding one of the guide spaces (15; 25; 35) between them.

3. Guiding device (1) as claimed in claim 2, wherein the anisotropic three-dimensional structure (8; 8'; 8") comprises a plurality of guide elements (10; 20; 30) protruding from the base surface (7), said guide elements (10; 20; 30) being adjacent to one another along the repeat axis (X) and each bearing one of the first guide surfaces (14; 24; 34) and one of the second guide surfaces (16; 26; 36), the first guide surface (14; 24; 34) of one of the guide elements (10; 20; 30) being disposed facing the second guide surface (16; 26; 36) of the adjacent guide element (10; 20; 30).

4. Guiding device (1) as claimed in claim 2 or 3, wherein the first (14; 24) and second guide surfaces (16; 26) of each pair are configured so that the direction of anisotropy (A1; A2) extends along the repeat axis (X).

5. Guiding device (1) as claimed in claim 4, wherein, in each of the pairs of guide surfaces, the first guide surface (14; 24) is configured to block movement of the cells (3) in a direction opposite the second guide surface (16; 26) and the second guide surface (16; 26) is configured to permit movement of the cells (3) in a direction opposite the first guide surface (14; 24) so that the direction of anisotropy (A1; A2) is oriented from the first guide surface (14; 24) towards the second guide surface (16; 26).

6. Guiding device (1) as claimed in claim 5, wherein the first guide surface (14; 24) is perpendicular to the repeat axis (X) and the second guide surface (16; 26) is spaced apart from the first guide surface (14; 24) along the repeat axis (X).

7. Guiding device (1) as claimed in claim 6, wherein the second guide surface (16) is perpendicular to the base surface (7) and has a concavity directed towards the first guide surface (14).

8. Guiding device (1) as claimed in claim 7, wherein the anisotropic three-dimensional structure (8) comprises a plurality of rows (10) of adjacent triangular projections along the repeat axis (X), each of said rows (10) comprising at least two projections (11) aligned along a transverse axis (Y) perpendicular to the repeat axis (X), each of the guide spaces comprising a substantially triangular cavity (15) with a base (17) formed on the first guide surface (14) of one of the rows (10) of triangular projections and an apex (18) formed on the second guide surface (16) of the adjacent row (10) of triangular projections.

9. Guiding device (1) as claimed in claim 6, wherein the first guide surface (24) is perpendicular to the repeat axis and the second guide surface (26) is inclined relative to a plane perpendicular to the base surface (7).

10. Guiding device (1) as claimed in claim 2 or 3, wherein the first (34) and second (36) guide surfaces of each pair are configured so that the direction of anisotropy (A3a, A3b) extends along a transverse axis (Y) perpendicular to the repeat axis (X).

11. Guiding device (1) as claimed in claim 10, wherein, in each of the pairs of guide surfaces, the first (34) and second (36) guide surfaces are configured to block movement of the cells (3) in one or the other of the directions of the repeat axis (X).

12. Guiding device (1) as claimed in claim 11, wherein the anisotropic three-dimensional structure (8") comprises a plurality of adjacent elongate projections (30) along the repeat axis (X), each of said elongate projections (30) extending along the transverse axis (Y), each of the guide spaces comprising a groove (35) between the first guide surface (34) on one of the elongate projections (30) and the second guide surface (36) of the adjacent elongate projection (30).

13. Guiding device (1) as claimed in any one of claims 2 to 12, wherein the guide space (15; 25; 35) has a maximum dimension, measured between the first (14; 24; 34) and second guide surfaces (16; 26; 36), of less than 200 µm, preferably less than 100 µm, in particular substantially corresponding to a size of the cells (3), for example between 5 µm and 60 µm, preferably between 15 µm and 30 µm.

14. Guiding device (1) as claimed in any one of claims 2 to 13, wherein the guide space (15; 25; 35) has a depth of less than 200 µm, preferably less than 100 µm, in particular less than a size of the cells (3), for example less than 6 µm.

15. Guiding device (1) as claimed in any one of claims 1 to 14, wherein the substrate (5) is non-adhesive.

16. Guiding device (1) as claimed in claim 15, wherein the non-adhesive substrate (5) is made from a non-adhesive material such as a fluorinated polymer or a material rendered non-adhesive by a chemical treatment such as grafting molecules of polyethylene glycol (PEG).

17. Guiding device (1) as claimed in any one of claims 1 to 14, wherein the substrate (5) is adhesive.

18. Guiding device (1) as claimed in any one of claims 1 to 17, wherein the support surface (2) on which the cells (3) move is an artificial surface such as a cell culture surface (for example a gel), a glass slide, the interior of a microfluidic passage or a surface of the natural environment of said cells such as the surface of a living tissue or the surface of a wound.

19. Guiding device (1) as claimed in any one of claims 1 to 18, wherein the support surface (2) and the textured surface (6; 6'; 6") are spaced at a distance of between 0 µm and 10 µm, preferably between 3 µm and 6 µm.

20. Guiding device (1) as claimed in claim 19, wherein at least one of the surfaces selected from the support surface (2) and the textured surface (6; 6'; 6") comprises at least one additional protuberance enabling the distance between the support surface (2) and the textured surface (6; 6'; 6") to be controlled.

21. Guiding device (1) as claimed in claim 20, wherein the additional protuberance is provided in the form of a post with a diameter of between 100 µm and 500 µm and a height of less than 10 µm, preferably between 3 µm and 6 µm.

22. Guiding device (1) as claimed in any one of claims 1 to 21, in the form of a dressing, an implant, a prosthesis, a support for artificial tissues, a microfluidic passage, a lab-on-a-chip integrating passages, and preferably said guide device (1) is a dressing.

23. Method of guiding cell migration using a guide device (1) as claimed in any one of claims 1 to 22, said guiding method involving placing the cells (3) disposed on the support surface (2) in contact with the textured surface (6; 6'; 6") of the substrate (5) so that the cells (3) are confined between the support surface (2) and the textured surface (6; 6'; 6"), the cells moving in the direction of anisotropy (A1; A2; A3a, A3b).
